Europäisches Patentamt

European Patent Office

Office européen des brevets

(19)

(11) Veröffentlichungsnummer: **0 128 279**
**B1**

(12) EUROPÄISCHE PATENTSCHRIFT

(45) Veröffentlichungstag der Patentschrift:
25.01.89

(51) Int. Cl.⁴: **C 07 D 213/807**

(21) Anmeldenummer: **84103322.8**

(22) Anmeldetag: **26.03.84**

(54) Verfahren zur Herstellung von 6-Methylnicotinsäureester.

(30) Priorität: **10.05.83 CH 2542/83**

(43) Veröffentlichungstag der Anmeldung:
**19.12.84 Patentblatt 84/51**

(45) Bekanntmachung des Hinweises auf die Patenterteilung:
**25.01.89 Patentblatt 89/4**

(84) Benannte Vertragsstaaten:
**CH DE FR GB LI SE**

(56) Entgegenhaltungen:
**US-A-2 475 969**
**US-A-2 694 070**
**US-A-2 749 350**
**US-A-2 993 904**

(73) Patentinhaber: **LONZA AG, Gampel/Wallis (CH)**

(72) Erfinder: **Grayson, James Ian, Dr.,**
**Bürchnerstrasse 11, Visp (Kanton Wallis) (CH)**

(74) Vertreter: **von Füner, Alexander, Dr.,**
**Patentanwälte v. Füner, Ebbinghaus, Finck**
**Mariahilfplatz 2 & 3, D-8000 München 90 (DE)**

## Beschreibung

Die Erfindung betrifft ein Verfahren zur Herstellung von 6-Methylnicotinsäure durch Oxydation von 2-Methyl-5-ethylpyridin bei erhöhter Temperatur. Es ist bekannt, 6-Methyl-nicotinsäureester durch Oxydation von 2-Methyl-5-ethylpyridin herzustellen. Dabei wird als Oxydationsmittel entweder Kaliumpermanganat oder Salpetersäure verwendet.

Das Kaliumpermanganat-Verfahren [J. Org. Chem., 24 (1959), 1190] birgt den Nachteil in sich, dass grosse Mengen Wasser und Kaliumpermanganat und lange Reaktionszeiten benötigt werden. So wird pro kg Endprodukt 10,9 kg Kaliumpermanganat und 364 l Wasser gebraucht. Die Reaktionsdauer war ca. 5 Tage; die Ausbeute lag bei 69 %.

Nach CA, Vol. 45 (1951), 9055, wird 2-Methyl-5-ethylpyridin mit Salpetersäure in Gegenwart von Ammoniumvanadat in die 6-Methylnicotinsäure übergeführt, letztere als Kupfersalz isoliert. Die Reaktionsdauer beträgt 3 Tage; die Ausbeute 51 %.

In der US-PS 29 91 285 wird vorstehende Methode modifiziert und die Isolierung der 6-Methylnicotinsäure erfolgt über Ionenaustauscher. In diesem Fall beträgt die Ausbeute 46 %.

Als Katalysator für die Salpetersäureoxydation wurde auch schon $SeO_2$ unter Lufteinleiten beschrieben (US-PS-2 749 350). Die Isolierung erfolgte als Kupfersalz in einer Ausbeute von 68 %. Nach Zersetzung des Kupfersalzes lag die Ausbeute an 6-Methylnicotinsäure bei 47 %.

Die Salpetersäureoxydation wurde auch schon unter Druck bei Temperaturen von 130 bis 180°C durchgeführt (US-PS-2 993 904). Dabei wurden nur äusserst niedrige Ausbeuten an 6-Methylnicotinsäure erzielt.

Ziel der Erfindung ist es, die Nachteile, die die bekannten Verfahren aufweisen, zu beseitigen und ein technisch durchführbares Verfahren zu finden. Erreicht wird dies durch ein Verfahren, wie es im Patentanspruch 1 beansprucht wird.

Beim Verfahren der Erfindung wird zunächst das 2-Methyl-5-ethyl-pyridin (1 Mol) mit 1 bis 5 Mol, vorzugsweise 2 bis 4 Mol, einer 20 bis 100-%-igen Schwefelsäure bei Temperaturen von 0 bis 100°C gemischt. Dieses Gemisch wird auf 140 bis 225°C, vorzugsweise auf 150 bis 170°C, aufgeheizt. Bei Verwendung einer Schwefelsäure mit einer Konzentration von weniger als 100 % wird die entsprechende Menge Wasser abdestilliert. In dieses Gemisch wird Salpetersäure mit einer Konzentration von 20 bis 70 %, in einer Menge von 3 bis 10 Mol, vorzugsweise 6 bis 9 Mol, pro Mol 2-Methyl-5-ethylpyridin so zudosiert, dass das entstehende Wasser und/oder die verdünnte Salpetersäure laufend abdestilliert. Dies dauert 2 bis 11 Stunden, normalerweise 5 bis 7 Stunden.

Ist die Zugabe der Salpetersäure zu Ende, wird das Reaktionsgemisch noch so lange auf Reaktionstemperatur oder höher, z. B. bis 190°C gehalten, bis alles Wasser und alle Salpetersäure abdestilliert ist. Normalerweise dauert dies 15 bis 30 Minuten.

Die Oxydation kann mit oder ohne Zugabe von Katalysatoren, wie Ammoniumvanadat, Zinnchlorid, Kobaltacetat, durchgeführt werden. Werden Katalysatoren angewendet, so werden Mengen eingesetzt, die kleiner sind als 5 g pro Mol 2-Methyl-5-ethylpyridin, vorzugsweise kleiner als 2 g.

Die während der Reaktion abgezogene verdünnte Salpetersäure kann wieder aufkonzentriert und in den Prozess zurückgeführt werden.

Nach Abdestillieren der Salpetersäure und des Wassers wird das im Reaktionsgefäss zurückbleibende Gemisch, das zur Hauptsache aus 2-Methyl-5-ethylpyridin, 6-Methylnicotinsäure und Isocinchomeronsäure, alle als Schwefelsäuresalze, besteht, mit einem Alkohol versetzt und auf Temperaturen von 25°C bis zum Rückflusspunkt des Alkohols aufgeheizt. Es wird die Temperatur solange beibehalten, bis die organischen Säuren verestert sind.

Je nach gewünschtem Ester werden die Alkohole ausgewählt. Es können primäre, sekundäre oder tertiäre Alkohole einge setzt werden. So z. B. Methanol, Ethanol, Isopropanol, n-Butanol, t-Butanol.

Die Menge einzusetzenden Alkohols liegt bei mindestens 1 Mol pro Mol eingesetztes 2-Methyl-5-ethylpyridin, vorzugsweise 5 bis 10 Mol.

Die nach der Veresterung vorliegende Lösung wird eingedampft, neutralisiert und extrahiert. Zur Neutralisation werden z. B. Ammoniak, Natriumhydroxid und Kaliumhydroxid verwendet. Zur Extraktion werden zweckmässig die folgenden Lösungsmittel verwendet: Methylenchlorid, Chloroform, Ether, Toluol. Nach fraktionierter Destillation der Extrakte erhält man die einzelnen Produkte 2-Methyl-5-ethylpyridin, 6-Methylnicotinsäureester und Isocinchomeronsäurediester, wobei die einzelnen Ausbeuten bei etwa 10 bis 12 %, 65 bis 70 % und 3 bis 5 % liegen.

## Beispiel 1

In einen 1,5-Liter-Sulfierkolben wurden 225,0 g (2,26 Mol) 96-%-ige Schwefelsäure und 0,5 g (4,3 mMol) Ammoniumvanadat vorgelegt. Der Kolben wurde gekühlt und 92,7 g (0,75 Mol) 98-%-iges 2-Methyl-5-ethylpyridin innerhalb 25 Minuten zugetropft. Das gerührte Reaktionsgemisch wurde auf 160°C aufgeheizt und während 5 Std. 30 Min. wurden 600,0 g (6,2 Mol) 65-%-ige Salpetersäure zugetropft, so dass die Reaktionstemperatur zwischen 155 und 160°C blieb. Während dieser Zeit wurden 445,0 g Salpetersäure kontinuierlich abdestilliert. Das Destillat hatte einen Gehalt von 46,9 %, was einem Verbrauch während der Reaktion von 2,89 Mol Salpetersäure entspricht. Nach Ende der Salpetersäurezugabe wurde die Reaktion während 15 Min. auf 190°C erhitzt, um unreagierte Salpetersäure abzudestillieren. Das Reaktionsgemisch wurde anschliessend unter 100°C gekühlt und in 300 ml Ethanol gegossen. Der Reaktionskolben wurde mit 100 ml Ethanol nachgewaschen und die ganze Ethanollösung wurde während 6 Std. unter Rückfluss gekocht. Das Ethanol wurde abdestilliert und der Rückstand auf 300 g Eis gegossen und mit 25-%-iger Ammoniaklösung neutralisiert. Das Produkt wurde mit 3 mal 100 ml Methylenchlorid extrahiert. Die Methylenchloridextrakte wurde eingedampft und der Rückstand mittels Gaschromatographie analysiert.

Die GC-Analyse ergab 11,8 % 2-Methyl-5-ethylpyridin, 67,9 % 6-Methylnicotinsäureethylester und 3,5 % Isocinchomeronsäurediethylester.

Sämtliche gaschromatographischen Analysen wurden unter Verwendung eines internen Standards sowie unter Berücksichtigung von Flächenkorrekturfaktoren durchgeführt.

Die Rohprodukte aus 5 solchen Versuchen wurden destilliert, wobei 57,1 g 2-Methyl-5-ethylpyridin (12,6 %) zuerst destillierte, gefolgt von 409,9 g 98,3-%-igem 6-Methylnicotinsäure-ethylester von Kp. 121 bis 125°C bei 20 mm Hg (65,0 % Ausbeute). Die Selektivität an 6-Methylnicotinsäureethylester betrug 74,4 %.

### Beispiel 2

Beim Beispiel 2 wurde prinzipiell auf gleiche Weise gearbeitet wie beim Beispiel 1, ausser dass anstelle von Ethanol Methanol gebraucht wurde.

Die Rohprodukte aus 5 Versuchen wurden destilliert, woboi 53,5 g 2-Methyl-5-ethylpyridin (11,7 %) zuerst destillierte, gefolgt von 404,7 g 97,6-%-igem 6-Methylnicotinsäuremethylester mit Kp. von 112 bis 115°C bei 20 mm Hg (69,7 % Ausbeute).

Die Selektivität an 6-Methylnicotinsäuremethylester betrug 78,9 %.

### Beispiel 3

Es wurde prinzipiell auf die gleiche Weise gearbeitet wie beim Beispiel 1. 630,0 g 65-%-ige Salpetersäure wurden zugegeben und die Reaktionstemperatur war 160 bis 165°C. Das Produkt wurde mit 400 ml Isopropanol während 18 Std. bei Rückflusstemperatur verestert.

Der Rohester wurde destilliert, wobei 5,0 g 2-Methyl-5-ethylpyridin (5,6 %) zuerst destillierte, gefolgt von 77,5 g 97,2-%-igem 6-Methylnikotinsäureisopropylester mit Kp. von 70 bis 72°C bei 0,6 mm Hg (56,1 % Ausbeute).

Die Selektivität an 6-Methylnicotinsäureisopropylester betrug 59,4 %.

### Beispiel 4

Es wurde prinzipiell auf die gleiche Weise gearbeitet wie beim Beispiel 1. 630,0 g 65-%-ige Salpetersäure wurden zugegeben und die Reaktionstemperatur war 160 bis 165°C. Das Produkt wurde mit 400 ml n-Butanol während 18 Std. bei Rückflusstemperatur verestert.

Der Rohester wurde destilliert, wobei 5,7 g 2-Methyl-5-ethylpyridin (6,3 %) zuerst destillierte, gefolgt von 92,3 g 97,3-%-igem 6-Methylnicotinsäure-n-butylester mit Kp. von 95 bis 97°C bei 0,6 mm Hg (63,2 % Ausbeute).

Die Selektivität an 6-Methylnicotinsäure-n-butylester betrug 67,4 %.

### Beispiele 5 - 15

Bei den Beispielen 5 - 15 wurde prinzipiell auf die gleiche Weise gearbeitet wie beim Beispiel 1. Die Reaktionsbedingungen und die Ausbeuten (nach gaschromatographischer Analyse der Rohprodukte) werden in der nachfolgenden Tabelle zusammengestellt.

**TABELLE**

| Beispiel Nr. | Edukte 2-Methyl -5-ethyl- pyridin (g) | Schwefel- säure (g) | Ammonium- vanadat (g) | Salpeter- säure (g) | Reaktions- temperatur (°C) | Salpetersäure- Zudosierungs- zeit (Std.) | Veresterungs- zeit (Std.) |
|---|---|---|---|---|---|---|---|
| 5 | 92,7 | 200 | 1,0 | 582 | 150 - 155 | 6 | 18 |
| 6 | 92,7 | 200 | 0,5 | 679 | 180 - 185 | 5 | 18 |
| 7 | 92,5 | 250 | 1,8 | 582 | 145 - 150 | 5 1/2 | 18 |
| 8 | 92,7 | 225 | 3,6 | 630 | 150 - 155 | 5 1/2 | 18 |
| 9 | 92,7 | 225 | - | 679 | 165 - 170 | 6 | 18 |
| 10 | 92,7 | 250 | - | 630 | 190 - 195 | 5 1/2 | 60 |
| 11 | 92,7 | 300 | 0,3 | 582 | 165 - 170 | 5 | 18 |
| 12 | 92,7 | 225 | - | 291 | 175 - 180 | 2 | 18 |
| 13 | 92,7 | 225 | - | 654 | 175 - 180 | 11 | 18 |
| 14 | 92,7 | 112 | - | 291 | 220 - 225 | 2 | 18 |
| 15 | 92,7 | 225 | - | 654 | 220 - 225 | 4 1/4 | 18 |

| Beispiel Nr. | Ausbeute 2-Methyl-5- ethylpyridin (%) | 6-Methylnicotin- säureethyl- ester (%) | Isocinchomeron- säurediethyl- ester (%) | Selektivität an 6-Methylnicotin- säureethylester (%) |
|---|---|---|---|---|
| 5 | 8,1 | 69,9 | 4,3 | 76,1 |
| 6 | 0,1 | 62,0 | 14,3 | 62,1 |
| 7 | 6,4 | 69,4 | 5,1 | 72,6 |
| 8 | 0,3 | 63,5 | 10,4 | 63,7 |
| 9 | 25,0 | 57,7 | 2,8 | 76,9 |
| 10 | 7,9 | 61,7 | 9,8 | 67,0 |
| 11 | 14,2 | 63,9 | 2,6 | 74,5 |
| 12 | 48,9 | 35,8 | 3,9 | 70,0 |
| 13 | 1,0 | 67,6 | 12,4 | 68,3 |
| 14 | 28,5 | 27,5 | 6,9 | 38,5 |
| 15 | 10,9 | 37,3 | 19,7 | 41,9 |

**Beispiel 16**

In einem 1,5-Liter-Sutfierkolben wurden 150,0 g (1,5 Mol) 96-%-ige Schwefelsäure und 0,9 g (7,5 mMol) Ammoniumvanadat vorgelegt. Der Kolben wurde gekühlt und 92,7 g (0,75 Mol) 98-%-iges 2-Methyl-5-ethylpyridin innerhalb 20 Min. zugetropft. Das gerührte Reaktionsgemisch wurde auf 200°C aufgeheizt und während 5 Std. wurden 509 g (5,25 Mol) 65-%-ige Salpetersäure zugetropft, so dass die Reaktionstemperatur zwischen 200 und 205°C blieb. Während dieser Zeit wurde Salpetersäure kontinuierlich abdestilliert. Nach Ende der Salpetersäurezugabe wurde das Reaktionsgemisch 15 Min. bei 200°C geheizt und anschliessend auf 120°C gekühlt. 150 ml Wasser wurde zugegeben und dann das Reaktionsgemisch auf 150 ml Wasser gegossen. Der Reaktionskolben wurde mit 2 mal 100 ml Wasser gewaschen und die gesammelte Wasserfraktionen wurden bis 0°C gekühlt.

Es fielen 41,8 g Isocinchomeronsäure aus (33,3 % Ausbeute), die abfiltriert wurden.

Die Mutterlaugen wurden eingedampft und der Rückstand in 500 ml Ethanol gelöst. Die Lösung wurde während 18 Std. unter Rückfluss erhitzt und anschliessend wie in Beispiel 1 aufgearbeitet.

Die gaschromatographische Analyse des Roh-Estergemisches ergab 0,4 % 2-Methyl-5-ethylpyridin, 50,1 % 6-Methylnicotinsäureethylester und 3,2 % Isocinchomeronsäurediethylester.

Die Selektivität an 6-Methylnicotinsäureethylester betrug 50,3 %.

**Patentansprüche**

1. Verfahren zur Herstellung von 6-Methylnicotinsäureester durch Oxydation von 2-Methyl-5-ethylpyridin bei erhöhter Temperatur, dadurch gekennzeichnet, dass man 2-Methyl-5-ethylpyridin unter Kühlung mit mindestens der molaren Menge Schwefelsäure mischt, dieses Gemisch auf Temperaturen von 140 bis 225°C aufheizt und diesem erhitzten Gemisch mindestens 3 Mol Salpetersäure pro Mol Edukt in der Weise zusetzt, dass laufend Wasser und/oder verdünnte Salpetersäure abdestilliert, dieses Abdestillieren nach Zugabe der für die Oxydation benötigten Salpetersäure noch so lange weiterführt, bis das gesamte Wasser und die gesamte Salpetersäure entfernt sind, anschliessend das Reaktionsgemisch mit einem Alkohol versetzt und solange erwärmt, bis die im Reaktionsgemisch enthaltenen organischen Säuren verestert sind und aus diesem Estergemisch den 6-Methylnicotinsäureester in an sich bekannter Weise isoliert.

2. Verfahren nach Patentanspruch 1, dadurch gekennzeichnet, dass man pro Mol 2-Methyl-5-ethylpyridin 1 bis 5 Mol Schwefelsäure mit einer Konzentration von 20 bis 100 % verwendet.

3. Verfahren nach Patentanspruch 1 und 2, dadurch gekennzeichnet, dass man pro Mol 2-Methyl-5-ethylpyridin 3 bis 10 Mol Salpetersäure mit Konzentration von 20 bis 70 % verwendet.

4. Verfahren nach Patentanspruch 1 bis 3, dadurch gekennzeichnet, dass man die Oxydation in Gegenwart eines Oxydationskatalysators durchführt.

5. Verfahren nach Patentanspruch 1 bis 4, dadurch gekennzeichnet, dass man zur Isolierung des 6-Methylnicotinsäureesters das Estergemisch eindampft, die Schwefelsäure mit einer Base neutralisiert, die Ester mit einem organischen Lösungsmittel extrahiert und diesen Extrakt fraktioniert destilliert.

## Claims

1. Process for the preparation of 6-methylnicotinate by oxidation of 2-methyl-5-ethylpyridine at elevated temperature, characterised in that 2-methyl-5-ethylpyridine is mixed with at least the molar amount of sulphuric acid with cooling, this mixture is heated to temperatures of 140 to 225 C and to this heated mixture at least 3 moles of nitric acid per mole of educt is added in such a way that water and/or diluted nitric acid is distilled off continuously, after adding the nitric acid required for the oxidation, this distilling off process is continued until all of the water and all of the nitric acid has been removed, finally the reaction mixture is treated with an alcohol and heated until the organic acids contained in the reaction mixture are esterified and the 6-methyl-nicotinate is removed from this ester mixture in a manner known per se.

2. Process according to patent claim 1, characterised in that 1 to 5 moles of sulphuric acid having a concentration of 20 to 100 % are used per mole of 2-methyl-5-ethylpyridine.

3. Process according to patent claim 1 and 2, characterised in that 3 to 10 moles of nitric acid having a concentration of 20 to 70 % are used per mole of 2-methyl-5-ethylpyridine.

4. Process according to patent claim 1 to 3, characterised in that the oxidation is carried out in the presence of an oxidation catalyst.

5. Process according to patent claim 1 to 4, characterised in that the ester mixture is concentrated by evaporation in order to remove the 6-methylnicotinate, the sulphuric acid is neutralised with a base, the ester is extracted by means of an organic solvent and this extract is fractionally distilled.

## Revendications

1. Procédé de préparation d'esters de l'acide 6-méthylnicotinique par oxydation de la 2-méthyl-5-éthylpyridine à température élevée, caractérisé en ce qu'on mélange de la 2-méthyl-5-éthylpyridine, en refroidissant, avec au moins la quantité molaire d'acide sulfurique, on chauffe ce mélange à des températures de 140 à 225°C et on ajoute à ce mélange chauffé au moins 3 moles d'acide nitrique par mole de produit de départ, de telle manière qu'il s'élimine en continu par distillation de l'eau et/ou de l'acide nitrique dilué, on poursuit cette élimination par distillation après addition de l'acide nitrique nécessaire pour l'oxydation, jusqu'à ce que toute l'eau et tout l'acide nitrique aient été éliminés, puis on additionne le mélange réactionnel d'un alcool et on le chauffe jusqu'à ce que les acides organiques contenus dans le mélange réactionnel soient estérifiés, et on isole de ce mélange d'esters l'ester de l'acide 6-méthylnicotinique d'une manière connue en soit.

2. Procédé suivant la revendication 1, caractérisé en ce qu'on utilise, par mole de 2-méthyl-5-éthylpyridine, 1 à 5 moles d'un acide sulfurique ayant une concentration de 20 à 100 %.

3. Procédé suivant les revendications 1 et 2, caractérisé en ce qu on utilise par mole de 2-méthyl-5-éthylpyridine, 3 à 10 moles d'un acide nitrique ayant une concentration de 20 à 70 %.

4. Procédé suivant les revendications 1 à 3, caractérisé en ce qu'on effectue l'oxydation en présence d'un catalyseur d'oxydation.

5. Procédé suivant les revendications 1 à 4 caractérisé en ce que, pour l'isolement de l'ester de l'acide 6-méthylnicotinique, on fait évaporer le mélange d'esters, on neutralise l'acide sulfurique avec une base, on extrait les esters avec un solvant organique et on soumet cet extrait à une distillation fractionnée.